# EUROPEAN PATENT APPLICATION

(11) **EP 2 258 434 A1**
(43) Date of publication of application: **08.12.2010**
(21) Application number: 10180784.0
(22) Date of filing: 28.06.2002
(51) Int. Cl.: A61M 25/00

(54) **A method of producing a catheter and a catheter**

(30) Priority: 29.06.2001 DK 200101041; 29.06.2001 US 893514; 24.09.2001 DK 200101386; 13.12.2001 DK 200101869; 13.12.2001 DK 200101870; 27.12.2001 US 26819; 17.04.2002 DK 200200569; 17.04.2002 DK 200200570; 13.06.2002 DK 200200895
(62) Divisional of application: 08152518.0
(71) Applicant: Coloplast A/S, 3050 Humlebaek (DK)
(72) Inventor: Tanghoej, Allan, 2980, Kokkedal (DK); Jensen, Lars Boegelund, 2610, Roedovre (DK)

(57) **Abstract**

The present invention is a method of producing a medical catheter having a catheter body part and a catheter tip in one integrated part by injection moulding and a catheter produced by the method. The invention is in particular concerned with the use of multi-component moulding technique for producing a catheter, e.g. a catheter made from two materials with different characteristics. Such a catheter may be used for urinary catheterisation and for similar purposes of draining a bodily fluid.

## Description

### Field of the invention

The present invention relates to a method of producing a medical catheter. In particular, the invention relates to a method wherein a catheter is produced by solidifying a fluid catheter material in a mould.

### Background of the invention

In general, medical catheters are used for draining bodily fluids such as blood and urine. A catheter for medical use is typically provided with a tubular oblong catheter body part made from a piece of flexible medical hose with an internal conduit, e.g. a PVC or PU hose having a substantially circular outer and inner cross-sectional shape. In an insertable end thereof, the catheter forms one or more openings through which fluid can drain from a bodily cavity and into the tubular body. In order to ease the insertion and to avoid injuring the bodily tissue when the catheter is inserted into a body opening and guided through a bodily canal, e.g. the urethra or a blood vessel, the insertable end is normally provided with a smoothly rounded tip. In the case of most catheters, the tip is formed by heating and melting an end part of the medical hose until its conduit seals. Even though the existing catheters are formed with a tip providing an acceptably safe and comfortable insertion, it is a desire to further shape a larger and more curved tip of the inserted part of the catheter since this may allow for an easier and safer insertion. However, the present technique of forming the tip of a medial hose does not support in making such a tip. As an alternative, some catheters are made from a medical hose with a glued-on catheter tip. This solution allows for a catheter with a larger and more curved tip, but the additional process step of providing a tip part and gluing the part to the medical hose implies additional production costs. Moreover, there is potential risk that the tip falls off during the catheterisation and thus remains inside the body cavity, e.g. in the bladder. For economical and for safety reasons, the on-glued catheter tip is therefore undesired.

When the tip has been formed, a number of draining holes are normally drilled or punched radially into the hose in the vicinity of the tip. In order to avoid that the holes damage the bodily tissue during the insertion of the catheter, the edges of the holes must be smoothly rounded. Accordingly, the process of making the holes is time-consuming and expensive.

At its opposite end, the oblong catheter body is formed with an opening allowing the fluid to drain out of the catheter and into an appropriate place of disposal. In this opposite end, most catheters are provided with a connector part. The connector part allows the catheter to be connected e.g. to a bag for collecting the fluid. The connector part is normally formed by adhesively bonding a funnel-shaped member to the medical hose. Once again, the additional process of gluing a separate member onto the hose is cost-inefficient and implies a larger percentage of defect products.

### Description of the invention

It is an object of the present invention to overcome the above described problems by providing a method of producing a catheter, said method comprising the steps of:
- injecting a fluid catheter material into a mould formed to define an insertable catheter tip and a catheter body in one part, and subsequently
- solidifying the material therein.

The injection may take place in a regular machine for injection moulding. Depending upon the size and length of the catheter, the injection pressure may be in the range of 500-1500 bar, such as 750-1250 bar such as in the size of 1000 bar.

The injection moulding process is in particular suitable for relatively short catheters, i.e. catheters which are in the range of 50-90 mm., such as in the range of 55-85 mm., such as in the range of 60-80 mm. such as with a length in the size of 70 mm. which length has been found to be a suitable insertable length for most female individuals. For male individuals, catheter sections may preferably be provided in a length in the range of 180-250 mm., such as in the range of 190-240 mm., such as in the range of 200-230 mm. such as in the size of 220 mm.

The catheter may further comprise connection means for connecting the proximal insertion section to a further catheter section or to a urinary collection bag. The connector part may be made from the same material as the proximal insertion section, whereby, at the step of forming the proximal insertion section, the proximal insertion section and the connector part may be formed substantially simultaneously. Alternatively, the connector part may be made from a material different from the material of the proximal insertion section, whereby the connector part and the proximal insertion section are formed in distinct process steps, for example in a multi-component injection moulding process.

The mould could be formed to define the body part as an oblong hollow, tubular part with an internal conduit of a size allowing bodily fluid to be drained through the catheter body. As an alternative, the body part could be provided in the form of an oblong solid kernel with one or more vanes extending radially from the kernel and along the entire length thereof. The vanes thus define a number of draining passages for draining urine between the kernel and a bodily draining passage, e.g. the urethra.

In order to allow the bodily fluid, e.g. urine from the bladder, to enter the hollow, tubular body part of the catheter, the mould could be formed to define at least one draining hole in the vicinity of the tip.

A connector part may be provided for connecting the catheter to a hose for extending the length of the catheter or for connecting the catheter to disposal means, e.g. to a urinary collection bag. The connector section could preferably be made in one part with the catheter body and the tip. The connector part may be made from the same material as the proximal insertion section and preferably substantially simultaneous therewith, e.g. during the same injection step. Alternatively, the connector part may be made from a material different from the material of the proximal insertion section. The connector part and the proximal insertion section are thus formed in distinct process steps, for example in a multi-component injection moulding process. The connector part may also be arranged as a separate component in the injection mould before the injection of the catheter material so that the connector part is moulded into engagement with the catheter body part during the injection moulding of the body part of the catheter. In a similar manner, a catheter tip may be arranged as a separate component in the mould and, during the injection moulding of the body part of the catheter, be moulded into the catheter. In a similar manner, additional components may be arranged in the mould prior to the injection moulding of the catheter body. As an example, one or more ring-shaped coloured members may be arranged for the purpose of visualising a certain length of the catheter, e.g. for visualising the intended insertable length. As another example, one or more objects made of a material, which improves the visualisation of the catheter in an x-ray or ultra-sound image may be arranged in the mould prior to the injection moulding of the catheter body.

In order to allow a user of the catheter to get a better grip, the catheter may be provided with means for attaching the catheter to peripheral articles such as a hand-grip for firmly gripping the catheter. The means for attaching the catheter to peripheral articles could be an out or inwardly extending bulge. As a further option, the catheter could be formed in one piece with means for handling the catheter during the insertion, e.g. a handle part which supports for a firm hand grip. As an example, the catheter could be provided in a diameter allowing for insertion into the urethra and with a catheter section not adapted for insertion and provided in a much larger diameter allowing a firm hand grip in the catheter.

Depending upon the type of catheterisation, the bodily fluid is typically drained either into a place of disposal e.g. into a toilet or into a reservoir or container for collecting the fluid. Accordingly, the mould could be formed to further define a reservoir for collecting the bodily fluids. The reservoir could be a plastic bag moulded in one piece with the catheter, e.g. by a combined injection and blow moulding process.

In order to fixate the catheter in the bodily passage, the catheter could be provided with a balloon in the vicinity of the inserted tip. The balloon could be moulded into one piece with the catheter.

Sometimes, it is desired that different parts of the catheter is provided with different characteristics. As an example, it may be desired for a urinary catheter that the insertable part is relatively soft and flexible so that the catheter can pass through the curved passage of the urethra. On the other hand, those parts of the catheter which is not adapted for insertion into the urethra may preferably be relatively less flexible, thus allowing an easier grip and allowing the inserted part of the catheter to be manipulated via the not inserted part.

Similarly, it may often be desired that the outer surface of the catheter is provided in a low-frictional material supporting an easier and more comfortable insertion of the catheter into the urinary canal. Accordingly, it may be desired to provide a surface layer of the catheter in a low-friction material such as FEP, PTFE or in a hydrophilic material such as polyvinylidone while the remaining part of the catheter is provided in a stronger and more durable material such as a thermoplastic elastomeric material, other thermoplastic materials, curable elastomeric materials, polyamide resins or elastomers or any mixture thereof, i.e. the group may comprise materials like, PVC, PU, PE, latex, and/or Kraton^{™}.

As an example, the mould may be coated with a hydrophilic material prior to the injection of a thermoplastic elastomeric material into the mould. As an alternative, series of injections of one or more types of thermoplastic elastomeric materials into the mould may take place. As an example, a hydrophilic material or a similar low frictional material such as siloxane, FEP etc. may firstly be injected to form an outer layer of the catheter. Subsequently, one or more types of materials, e.g. materials with different characteristics, are injected in one or more injection cycles in order to form the rest of the catheter.

As an advantage of the injection moulding process, the surface of the catheter may easily be provided with a surface roughness defining anchoring points for adhering a low frictional material to the surface of the catheter, e.g. anchoring points for bonding a FEP or PTFE (Teflon^{™}) coating to the surface.

When injecting the material in more than one injection cycle, a catheter either comprising multiple layers or laminates or more individual sections in the length, is formed. Due to the laminated structure, the laminated structure may be more durable towards mechanical stress. Due to the individual sections in the length, a first part of the catheter, e.g. the part of the catheter which is intended to be inserted into the urethra or similar body canal, may be made in a softer material than the remaining catheter or it may be made in another colour or with another slipperiness, i.e. more slippery than the parts not intended to be inserted.

The different characteristics may relate to the softness of the material after solidification, the colour of the material, the strength of the material, the slipperiness of the material after solidification or to the ability of the solidified material to absorb liquid substances.

As an example, the part of the catheter which is adapted for insertion into the body canal, may be coloured in a colour different from the colour of the other parts of the catheter. In that way, the user can easily avoid touching the insertable part of the catheter.

In order to reduce the resistance against insertion into the body canal, the radial size of the tip may be larger than the radial size of the rest of the catheter. As an example, the tip may be formed into an onion-shaped or bulbous knob or the tip may be formed with a conical shape. Preferably, the tip is provided with a first end part, fronting the body canal opening during the insertion and provided with a narrow radial size. From this end part, the tip widens out in an intermediate part until the radial size exceeds the radial size of the catheter body part. Between the intermediate part of the tip and the catheter body part, the radial size slopes down in a second end part until the radial size of the catheter body part.

Inlet openings for draining bodily fluid from a body cavity and into the catheter may be provided either in the first end part, in the intermediate part or in the second end part or in more than one part or even en all of the parts. As an example, the tip may be formed in with a plurality of small holes in the tip.

According to a second aspect, the present invention relates to a catheter formed by the above described method and comprising the above described features.

### Brief description of the drawings

Preferred embodiments of the invention will now be described in details with reference to the drawing in which:
Fig. 1 shows an injection-moulded catheter with a tip and a connector,
Fig. 2 shows a bulbous catheter tip,
Fig. 3 shows a conical catheter tip,
Fig. 4 shows a spherical catheter tip,
Fig. 5 shows a catheter with a reservoir for collecting bodily fluids, and
Fig. 6 shows a catheter in the form of an oblong solid kernel with a plurality of vanes extending radially from the kernel and along the entire length thereof.

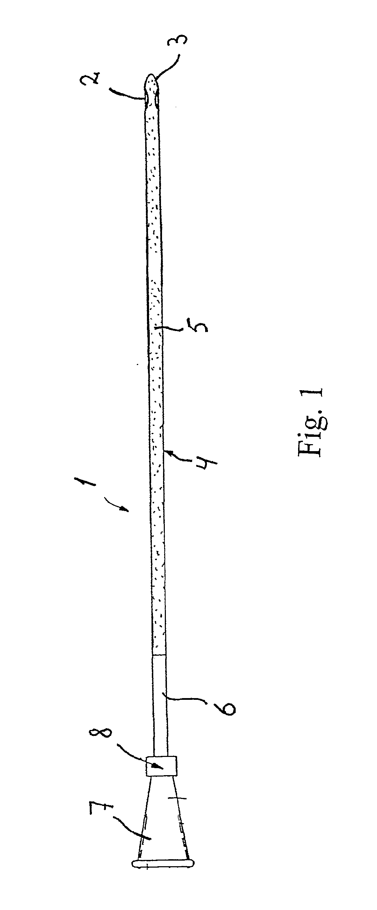

Referring to Fig. 1, the invention relates to a method of producing a catheter 1 by injecting a fluid catheter material into a mould formed to define an insertable catheter tip 3 and a catheter body 4 and subsequently solidifying the material therein. As shown, a catheter connector section 7 may be formed in one part with the catheter body. Furthermore, means 8 for attaching the catheter to peripheral articles for easing the handling of the catheter could be formed in one part with the catheter body during the moulding process.

In the insertable end, i.e. in the vicinity of the tip, a draining hole 2 allows bodily fluids to flow from a body cavity and into an internal conduit of the catheter. The internal conduit transports the fluid to the connector part where the fluid can be disposed into a bag or into a place of disposal.

The catheter of Fig. 1, is on its insertable part provided with a friction reducing material 5 and on its non-insertable part with a highly frictional material 6. The friction reducing material supports in a safe and easy insertion of the catheter into a body canal such as the urethra and the highly frictional material supports an easier grip in the non-inserted part and thus supports for easier manipulation of the catheter. The characteristics of the materials 5 and 6, respectively, could also relate to different colours indicating to the user which part of the catheter is intended to be inserted. The characteristics could also relate to different resiliency of the insertable part versus the non-insertable part e.g. allowing the inserted part easily to follow a curved body canal, e.g. for passing through the urethra around the prostate.

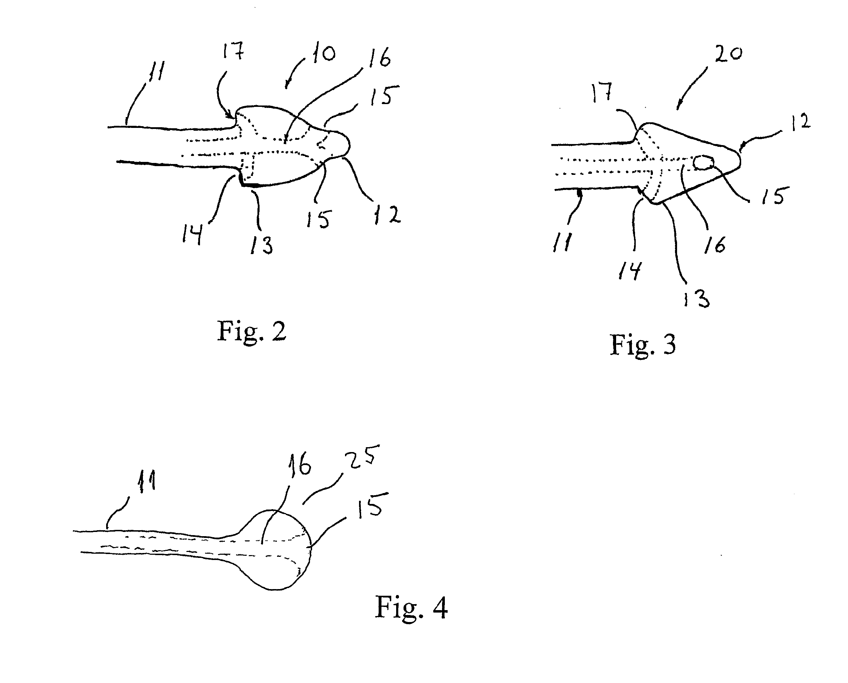

Figs. 2, 3 and 4 shows three different alternative tip shapes, i.e. a bulbous tip 10 of Fig. 2, a conical tip 20 of Fig. 3 and a spherical tip 25 of Fig. 4. The tip is provided in the inserted end of the catheter body 11 in one part with the body and during the moulding process. The tip is formed with a first end part 12 with a radial size which is smaller than the radial size of the catheter body, an intermediate part 13 with a radial size which is larger than the radial size of the catheter and a second end part 14 wherein the size slopes down to the radial size of the catheter. In order to allow bodily fluids to drain into an internal conduit of the catheter, draining holes are provided in one or more of the parts of the tip. In Figs. 2 and 3, draining holes 15 are provided in the first end part and draining holes 17 are provided in the second end part. The tip is internally moulded with a conduit 16 for guiding the fluid to the body part of the catheter. As shown in Fig. 4, a draining hole or holes may be provided in the tip, so that the opening point in the axial direction of the catheter body. In that case it is important that edges of the hole or holes are smoothly rounded in order not to injure the body canal.

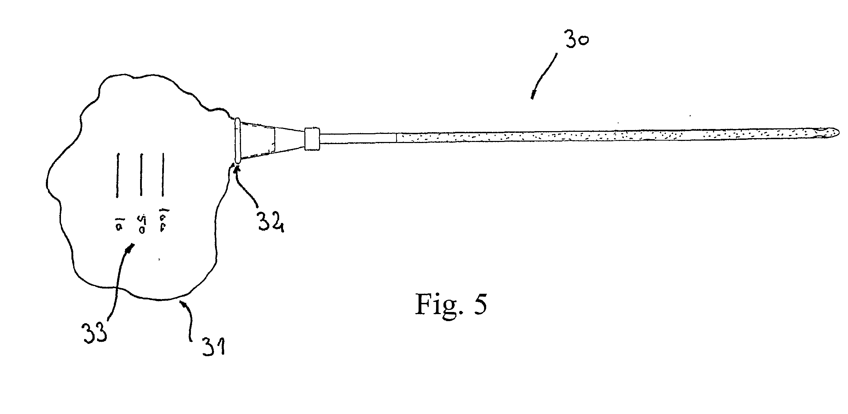

Fig. 5 shows a catheter 30 and a reservoir 31 formed by moulding in one part for collecting the bodily fluids, e.g. blood or urine. The reservoir could be formed during the injection moulding process by combining the injection moulding process with blow moulding. During this process, pressurised gas is used for expanding the reservoir part of the catheter product into a plastic bag or plastic bottle shaped item. The reservoir may, during the moulding process, be provided with filling level indication marks 33 and may internally be provided with a hydrophilic material for the conversion of a liquid substance into a substantially solid or gel-like substance.

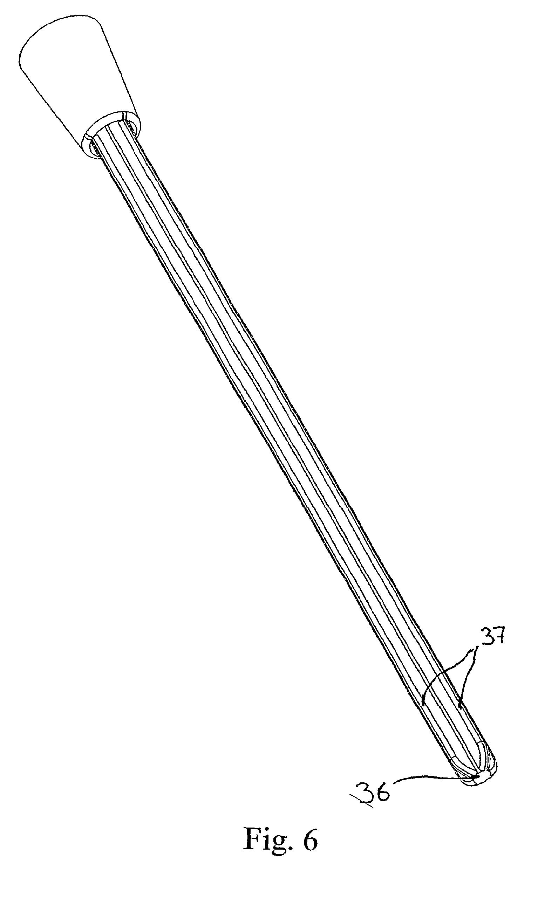

Fig. 6 shows catheter in the form of a solid kernel 36 with a plurality of radially extending vanes 37. The vanes form a number of draining passages for draining urine between the kernel and a bodily draining passage, e.g. the urethra.

### EMBODIMENTS

1. A method for producing a catheter said method comprising the steps of:
   - injecting a fluid catheter material into a mould formed to define an insertable catheter tip and a catheter body in one part, and subsequently
   - solidifying the material therein.
2. A method according to embodiment 1, wherein the mould is formed to define the body part with an internal conduit of a size allowing bodily fluid to be drained through the catheter body.
3. A method according to embodiment 1 or 2, wherein the mould is formed to define at least one draining hole in the vicinity of the tip.
4. A method according to any of embodiments 1-3, wherein the mould is formed to further define a connector section in one part with the catheter body and the tip for connection of the catheter to a place of disposal.
5. A method according to any of the preceding embodiments, wherein the mould is formed to further define means for attaching the catheter to peripheral articles in one part with the catheter body and/or the connector.
6. A method according to any of the preceding embodiments, wherein the mould is formed to further define means for handling the catheter during the insertion.
7. A method according to any of the preceding embodiments, wherein the mould is formed to further define a reservoir for collecting the bodily fluids.
8. A method according to any of the preceding embodiments, wherein the mould is formed to further define means for fixating the catheter in the bodily cavity.
9. A method according to any of the preceding embodiments, wherein different catheter materials are injected into the mould.
10. A method according to any of the preceding embodiments, wherein the catheter material is injected into the mould in more than one injection cycle.
11. A method according to embodiment 10, wherein catheter materials with different characteristics are in injected in each injection cycle.
12. A method according to embodiment 11, wherein the characteristics relates to the softness of the material after solidification.
13. A method according to embodiment 11 or 12, wherein the characteristics relates to the colour of the material.
14. A method according to any of embodiments 11-13, wherein the characteristics relates to the strength of the material.
15. A method according to any of embodiments 11-14, wherein the characteristics relates to the slipperiness of the material after solidification.
16. A method according to any of embodiments 11-15, wherein the characteristics relates to the ability of the solidified material to absorb liquid substances.
17. A method according to any of the preceding embodiments, wherein the mould is formed to define a tip with a larger radial size than the catheter body part.
18. A method according to any of the preceding embodiments, wherein the mould is formed to define a tip with a conical shape.
19. A method according to any of embodiments 1-17, wherein the mould is formed to define a tip with a bulbous shape.
20. An injection moulded catheter with a tip.
21. A catheter according to embodiment 20, comprising an internal conduit of a size allowing bodily fluid to be drained through the catheter body
22. A catheter according to embodiment 20, comprising at least one draining hole in the vicinity of the tip.
23. A catheter according to embodiment 22, comprising connection means in one part with the catheter body and the tip for connection of the catheter to a place of disposal.
24. A catheter according to any of embodiments 20-23, comprising means for attaching the catheter to peripheral articles in one part with the catheter body and/or the connector.
25. A catheter according to any of embodiments 20-24, comprising means for handling the catheter during the insertion.
26. A catheter according to any of embodiments 20-25, comprising a reservoir for collecting the bodily fluids.
27. A catheter according to any of embodiments 20-26, comprising means for fixating the catheter in the bodily cavity.
28. A catheter according to any of embodiments 20-27, made from more than one distinct catheter material
29. A catheter according to embodiment 28, wherein the characteristics of one of the materials makes the material more slippery during insertion into a body canal than the other material.
30. A catheter according to embodiment 28 or 29, wherein the characteristics of one of the materials makes the material softer than the other material.
31. A catheter according to any of embodiments 28-30, wherein the characteristics of one of the materials makes the material more easy to detect on an x-ray or an ultrasonic image than the other material.
32. A catheter according to any of embodiments 28-31, wherein at least one of the materials is selected from the group consisting of: thermoplastic elastomeric materials, other thermoplastic materials, curable elastomeric materials, polyamide resins or elastomers or any mixture thereof.
33. A catheter according to any of embodiments 28-32, wherein at least one of the materials is a hydrophobic polymer material.
34. A catheter according to any of embodiments 28-33, wherein at least one of the materials is a hydrophilic polymer material.
35. A catheter according to any of embodiments 29-31, wherein the material which is more slippery, forms an outer surface of an insertable part of the catheter.

## Claims

1. An injection moulded catheter having an insertable catheter tip and comprising a catheter body part having an internal conduit of a size allowing bodily fluid to be drained through the catheter body part, **characterized in that** the tip is provided with a first end part fronting the body canal opening during the insertion and provided with a narrow radial size, and from the first end part the tip widens out in an intermediate part until the radial size exceeds the radial size of the catheter body part, and where in a second end part between the intermediate part of the tip and the catheter body part the radial size of the intermediate part slopes down to the radial size of the catheter body part.

2. A catheter according to claim 1, comprising at least one draining hole in the vicinity of the tip.

3. A catheter according to any of the preceding claims, wherein at least one draining hole is provided in one or more of the first end part, the intermediate part and the second end part of the tip.

4. A catheter according to any of the preceding claims, wherein the tip is internally moulded with a conduit for guiding the fluid to the body part of the catheter.

5. A catheter according to any of the preceding claims, wherein at least one draining hole is provided in the tip of the catheter, as the opening point in the axial direction of the catheter body.

6. A catheter according to any of the preceding claims, wherein the tip is provided with a plurality of draining holes in the tip.

7. A catheter according to any of the preceding claims, comprising connection means in one part with the catheter body and the tip for connection of the catheter to a place of disposal.

8. A catheter according to any of the preceding claims, made from more than one distinct catheter material

9. A catheter according to claim 8, wherein the characteristics of one of the materials make the material more slippery during insertion into a body canal than the other materials.

10. A catheter according to claim 8 or 9, wherein the characteristics of one of the materials make the material softer than the other materials.

11. A catheter according to any of claims 8-10, wherein the characteristics of one of the materials make the material more easy to detect on an x-ray or an ultrasonic image than the other materials.

12. A catheter according to any of claims 8-11, wherein at least one of the materials is selected from the group consisting of: thermoplastic elastomeric materials, other thermoplastic materials, curable elastomeric materials, polyamide resins or elastomers or any mixture thereof.

13. A catheter according to any of claims 8-12, wherein at least one of the materials is a hydrophobic polymer material.

14. A catheter according to any of claims 8-13, wherein at least one of the materials is a hydrophilic polymer material.

15. A catheter according to any of claims 8-11, wherein the material which is more slippery forms an outer surface of an insertable part of the catheter.
